# EUROPEAN PATENT APPLICATION

(11) **EP 4 814 016 A1**
(43) Date of publication of application: **30.09.2026**
(21) Application number: 25166737.4
(22) Date of filing: 27.03.2025
(51) Int. Cl.: C07K 16/28, C07K 16/30, A61P 35/04

(54) **ANTIBODY OR FRAGMENT THEREOF SPECIFICALLY BINDING TO GLYCOPROTEIN 100**

(71) Applicant: Eberhard Karls Universität Tübingen (Medizinische Fakultät), 72074 Tübingen (DE)
(72) Inventor: PENGWEI, Li, 72074 Tübingen (DE); ETTERLIN, Tamara, 8037 Zürich (CH); SINNBERG, Tobias, 72072 Tübingen (DE); FLATZ, Lukas, 3074 Muri bei Bern (CH)
(74) Representative: Witte, Weller & Partner Patentanwälte mbB

(57) **Abstract**

The present invention relates to an antibody or fragment thereof specifically binding to glycoprotein 100 (gp100), the use of an antibody or fragment thereof in the prophylaxis and/or treatment of cancer, preferably gp100-expressing tumors, highly preferably melanoma, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of cancer, preferably gp100-expressing tumors, highly preferably melanoma, in a living being, comprising the administration of a prophylactically and/or therapeutically effective amount of said antibody or fragment thereof or said pharmaceutical composition or said nucleic acid or said vector.

## Description

### FIELD

The present invention relates to an antibody or fragment thereof specifically binding to glycoprotein 100 (gp100), the use of an antibody or fragment thereof in the prophylaxis and/or treatment of cancer, preferably gp100-expressing tumors, highly preferably melanoma, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of cancer, preferably gp100-expressing tumors, highly preferably melanoma, in a living being, comprising the administration of a prophylactically and/or therapeutically effective amount of said antibody or fragment thereof or said pharmaceutical composition or said nucleic acid or said vector.

### BACKGROUND

Melanoma, also redundantly known as malignant melanoma, is a type of cancer that develops from the pigment-producing cells known as melanocytes. Melanomas typically occur in the skin, but may rarely occur in the mouth, intestines, or eye. The primary cause of melanoma is ultraviolet light (UV) exposure in those with low levels of the skin pigment melanin.

According to GLOBOCAN 2020, malignant melanoma was worldwide diagnosed in 324,635 people and caused 57,043 deaths in 2020. The incidence is increasing to expected >500,000 in 2040. Melanoma is the most lethal skin cancer worldwide due to its high metastasis potential. While melanoma is curable through surgical intervention in the localized stage, a significantly worse mortality risk is observed with the occurrence of metastases. Patients with metastatic melanoma have, therefore, reduced survival compared with patients in earlier disease stages.

Chemotherapy drugs such as dacarbazine have been the backbone of metastatic melanoma treatment since FDA approval in 1975; however, its efficacy in terms of survival has never been proven in a randomized controlled trial.

Small-molecule targeted therapies work by blocking the genes involved in pathways for tumor proliferation and survival. The main treatments are BRAF, C-Kit and MEK inhibitors. These inhibitors work to inhibit the downstream pathways involved in cell proliferation and tumor development due to specific gene mutations. However, melanoma tumors can develop resistance during therapy which can make therapy no longer effective.

An overview of the current options for adjuvant therapy for melanoma can be found in Lao et al. (2022), Current state of adjuvant therapy for melanoma: less is more, or more is better? Am. Soc. Clin. Oncol. Educ Book 42, 1-7.

However, since the approval of treatment with so-called immune checkpoint inhibitors (ICls), over 50% of patients with newly diagnosed unresectable melanoma survive for five years or more. Despite advances in the development of innovative immunomodulatory therapies currently tested in clinical trials, a subset of patients treated with the established immunotherapies experience disease progression or severe toxicities, highlighting an ongoing medical and socio-economic need for novel therapeutic strategies.

### SUMMARY

Against this background, it is the object of the present invention to provide an alternative or new agent or compound with which the disadvantages of the prior art can be avoided or at least reduced. In particular, such an agent or compound is to be provided with which cancer, in particular melanoma, can be treated or even prevented in a targeted manner.

The problem underlying the invention is solved by the provision of an antibody or fragment thereof specifically binding to glycoprotein 100 (gp100), characterized in comprising
- as heavy chain variable domain a CDR3 region, said CDR3 region has an amino acid sequence being at least 90% identical to one selected from those in SEQ ID NO: 3, 19 or 35, and/or
- as light chain variable domain a CDR3 region, said CDR3 region has an amino acid sequence being at least 90% identical to one selected from those in SEQ ID NO: 6, 22, or 38.

The inventors have succeeded in isolating B cells expressing a B cell receptor against gp100 from the blood of melanoma patients treated with an ICI. The gene sequence of the B cell receptor was translated into antibody sequences. According to the inventors, recombinant antibodies with the sequences according to the invention mediate antibody-dependent cellular cytotoxicity (ADCC) against gp100-bearing tumor cells. The new antibodies therefore have a particularly high therapeutic potential in the prevention and treatment of gp100-expressing tumor cells, in particular melanoma cells, both in monotherapy and in combination with ICI.

The findings of the inventors were surprising and not expected. It is known that antibodies against melanocyte differentiation antigens (MDA), such as gp100, can be used as biomarkers for patients' responsiveness to ICI therapy and their chances of survival; see Fässler et al., Antibodies as biomarker candidates for response and survival to checkpoint inhibitors in melanoma patients. J. Immunother. Cancer, 2019. 7(1): p. 50, and de Joode et al., Suitability of tumor-associated antibodies as predictive biomarker for response to immune checkpoint inhibitors in patients with melanoma: a short report. J. Immunother. Cancer, 2023. 11(2). However, it was previously unknown that anti-gp100 antibodies themselves have tumor-destroying effects.

"Glycoprotein 100" (gp100) (human: Entrez 6490; Uniport P40967), also known as melanocyte protein PMEL, premelanosome protein (PMEL) or silver locus protein homolog (SILV), is a protein that in humans is encoded by the PMEL gene. gp100 is a 100 kDa, 661 amino acids long type I transmembrane glycoprotein that is expressed primarily in melanosomes, which are the melanin-producing organelles in melanocytes of pigment cells of the skin and eye, and in most malignant melanomas. This protein is involved in melanosome maturation, including melanogenesis, melanosome biogenesis, and melanin polymerization. gp100 is a melanoma antigen, i.e., a tumor-associated antigen.

In the context of the invention, the term "antibody" is intended to include any polypeptide chain-containing molecular structure with a specific shape that fits to and recognizes an epitope, where one or more non-covalent binding interactions may stabilize the complex between the molecular structure and the epitope. The term includes both polyclonal and monoclonal antibodies. The archetypal antibody molecule is the immunoglobulin, and all types of immunoglobulins are included, IgG, IgM, IgA, IgE, IgD, etc., from all sources, e.g., human, rodent, rabbit, cow, sheep, pig, dog, camelid, other mammals, chicken, other avians, etc., are considered to be "antibodies".

According to the invention, "CDR" refers to the complementary determining regions which, along with the framework regions (FR), are parts of the variable regions of the immunoglobulin and T cell receptor chains. While CDRs according to the invention are defined using the Kabat numbering scheme (see http://www.bioinf.org.uk/abs/info.html), functionally equivalent CDRs defined according to other established schemes, such as IMGT or Chothia, are also within the scope of the invention.

In an embodiment of the invention the antibody is a humanized antibody, i.e., an immunoglobulin or antibody that includes at least one humanized immunoglobulin or antibody chain (i.e., at least one humanized light or heavy chain). The term "humanized immunoglobulin chain" or "humanized antibody chain" (i.e., a "humanized immunoglobulin light chain" or "humanized immunoglobulin heavy chain") refers to an immunoglobulin or antibody chain (i.e., a light or heavy chain, respectively) having a variable region that includes a variable framework region substantially from a human immunoglobulin or antibody and CDRs (e.g., at least one CDR, preferably two CDRs, more preferably three CDRs) substantially from a non-human immunoglobulin or antibody, and further includes constant regions (e.g., at least one constant region or portion thereof, in the case of a light chain, and preferably three constant regions in the case of a heavy chain). The term "humanized variable region" (e.g., "humanized light chain variable region" or "humanized heavy chain variable region") refers to a variable region that includes a variable framework region substantially from a human immunoglobulin or antibody and CDRs substantially from a non-human immunoglobulin or antibody.

"Antibody fragments" comprise a portion of a full-length antibody, preferably the variable domain thereof, or at least the antigen binding site thereof, such as any of CDR3, CDR2 or CDR1. Examples of antibody fragments include diabodies, single-chain antibody molecules (scFv or scFab), and multispecific antibodies (e.g. bispecific) formed from antibody fragments.

The antibody or fragment thereof according to the invention can be produced by recombinant means, thereby resulting in a recombinant antibody or fragment. Thus, a recombinant antibody or antibody fragment is expressively encompassed. Methods for recombinant production are widely known in the state of the art and comprise protein expression in prokaryotic and eukaryotic cells with subsequent isolation of the antibody or fragment and usually purification to a pharmaceutically acceptable purity. For the expression of the antibodies or fragments as aforementioned in a host cell, nucleic acids encoding the respective light and/or heavy chains or fragments are inserted into expression vectors by standard methods. Expression is performed in appropriate prokaryotic or eukaryotic host cells like CHO cells, NSO cells, SP2/0 cells, HEK293 cells, COS cells, PER.C6 cells, yeast, or *E.coli* cells, and the antibody or fragment is recovered from the cells (supernatant or cells after lysis). General methods for recombinant production of antibodies are well-known in the state of the art and described, for example, in the review articles of Makrides, S.C. (1999), Protein Expr. Purif. 17, 183-202; Geisse et al. (1996), Protein Expr. Purif. 8, 271-282; Kaufman, R.J. (2000), Mol. Biotechnol. 16, 151-161; Werner, R.G. (1998), J. Drug Res. 48, 870-880.

As used herein, the term "binding" or "specifically binding" refers to the binding of the antibody or fragment thereof to an epitope of the antigen, e.g., with purified wild-type antigen in an *in vitro* assay such as a plasmon resonance assay (BlAcore, GE-Healthcare Uppsala, Sweden). Preferably, a specifically binding antibody or fragment does not exhibit significant cross-reactivity. An antibody or fragment thereof that "does not exhibit significant cross-reactivity" is one that will not appreciably bind to an undesirable entity, e.g., an undesirable proteinaceous entity. For example, an antibody or fragment that specifically bind to glycoprotein 100 (gp100) will appreciably bind gp100 but will not significantly react with non-gp100 proteins or peptides, e.g., non-gp100 proteins or peptides located in tumors. An antibody specific for a preferred epitope will, for example, not significantly cross-react with remote epitopes on the same protein or peptide. Specific binding can be determined according to any art-recognized means for determining such binding. Preferably, specific binding is determined according to Scatchard analysis and/or competitive binding assays. The affinity of the binding is defined by the terms ka (rate constant for the association of the antibody from the antibody/antigen complex), kD (dissociation constant), and KD (kD/ka). Binding or specifically binding means a binding affinity (KD) of 10⁻⁸ mol/l or less, preferably 10⁻⁹ M to 10⁻¹³ mol/l.

In an embodiment of the invention the antibody or fragment thereof is isolated. The term "isolated" means that the material is removed from its original environment (e.g., the natural environment, if it is naturally occurring). For example, a naturally occurring antibody or fragment thereof present in a living animal is not isolated, but the same antibody or fragment thereof, separated from some or all of the coexisting materials in the natural system, is isolated.

The inventors have recognized that, in one embodiment of the invention, the antibodies or fragments thereof need not necessarily be sequence identical with the indicated sequences, e.g., of the CDRs or CDR3 respectively. Specific and affine binding of the antibody or fragment is also possible if the binding regions are at least 90 % identical with the indicated specific sequences.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each amino acid or base in the Reference Sequence that does not have a corresponding aligned base or amino acid in the Compared Sequence and
(ii) each gap in the Reference Sequence and
(iii) each aligned amino acid or base in the Reference Sequence that is different from an aligned base or amino acid in the Compared Sequence, constitutes a difference and
(iv) the alignment has to start at position 1 of the aligned sequences;
and R is the number of amino acids or bases in the Reference Sequence over the length of the alignment with the Compared Sequence with any gap created in the Reference Sequence also being counted as an amino acid or base.

According to the invention, throughout the description and with respect to all embodiments, "at least 90 %" identity includes a sequence identity of 90, 91, 92, 93, 94, 95, 96, 97, 98, 99, and 100 %.

The antibody or fragment thereof disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from cellular material have been conventionally purified to electrophoretic homogeneity. Purification of starting material or natural material to at least one order of magnitude, preferably two or three orders, and more preferably four or five orders of magnitude is expressly contemplated. Furthermore, a claimed antibody or fragment thereof which has a purity of preferably 99.999 %, or at least 99.99 % or 99.9 %; and even desirably 99 % by weight or greater is expressly encompassed.

The antibody or fragment thereof according to the invention may be in "enriched form". As used herein, the term "enriched" means that the concentration of the material is at least about 2, 5, 10, 100, or 1000 times its natural concentration (for example), advantageously 0.01 %, by weight, preferably at least about 0.1 % by weight. Enriched preparations of about 0.5 %, 1 %, 5 %, 10 %, and 20 % by weight are also contemplated. The sequences, constructs, vectors, cells, and other materials comprising the present invention can advantageously be in enriched or isolated form.

The disclosed amino acid sequences have been identified by the inventors in such gp100-specific antibodies in the respective particular important CDR3 regions, in each case of the heavy and light chain, which are particularly suitable according to the invention to be applied prophylactically or therapeutically for the treatment of cancer, in particular melanoma.

In another embodiment of the invention said antibody or fragment thereof is further characterized in that
- the heavy chain variable domain comprises a CDR2 region, said CDR2 region has an amino acid sequence being at least 90% identical to one selected from those in SEQ ID NO: 2, 18 or 34, and/or
- the light chain variable domain comprises a CDR2 region, said CDR2 region has an amino acid sequence being at least 90% identical to one selected from those in SEQ ID NO: 5, 21 or 37.

In still another embodiment of the invention said antibody or fragment thereof is further characterized in that
- the heavy chain variable domain comprises a CDR1 region, said CDR1 region has an amino acid sequence being at least 90% identical to one selected from those in SEQ ID NO: 1, 17 or 33, and/or
- the light chain variable domain comprises a CDR1 region, said CDR1 region has an amino acid sequence being at least 90% identical to one selected from those in SEQ ID NO: 4, 20 or 36.

This measure provides the two further CDRs, i.e., CDR2 and CDR1, responsible for direct interaction with and binding to the antigen or epitope of the gp100 antigen. By including these additional CDRs the affinity, specificity and selectivity and thus also prophylactic and therapeutic suitability of the antibody according to the invention and fragment thereof are thus further increased.

Another embodiment of the invention provides the antibody or fragment thereof which comprises
- the heavy chain variable domains
   - CDR3 region having an amino acid sequence being at least 90% identical to said in SEQ ID NO: 3 and/or
   - CDR2 region having an amino acid sequence being at least 90% identical to said in SEQ ID NO: 2 and/or
   - CDR1 region having an amino acid sequence being at least 90% identical to said in SEQ ID NO: 1 and/or
- the light chain variable domains
   - CDR3 region having an amino acid sequence being at least 90% identical to said in SEQ ID NO: 6 and/or
   - CDR2 region having an amino acid sequence being at least 90% identical to said in SEQ ID NO: 5 and/or
   - CDR1 region having an amino acid sequence being at least 90% identical to said in SEQ ID NO: 4.

With this measure, the antigen-binding sites responsible for antigen binding and therapeutic efficacy of the particular affine and gp100-specific antibody designated by the inventors as 'clone A7' are made available. Said clone and, thus, the antibody and fragment thereof according to this embodiment, specifically and selectively bind to gp100. The antibody or fragment thereof according to this embodiment can be readily produced in a known manner.

Another embodiment of the invention provides the antibody or fragment thereof which comprising
- the heavy chain variable domains
   - CDR3 region having an amino acid sequence being at least 90% identical to said in SEQ ID NO: 19 and/or
   - CDR2 region having an amino acid sequence being at least 90% identical to said in SEQ ID NO: 18 and/or
   - CDR1 region having an amino acid sequence being at least 90% identical to said in SEQ ID NO: 17 and/or
- the light chain variable domains
   - CDR3 region having an amino acid sequence being at least 90% identical to said in SEQ ID NO: 22 and/or
   - CDR2 region having an amino acid sequence being at least 90% identical to said in SEQ ID NO: 21 and/or
   - CDR1 region having an amino acid sequence being at least 90% identical to said in SEQ ID NO: 20.

With this measure, the antigen-binding sites responsible for antigen binding and therapeutic efficacy of the particular affine and gp100-specific antibody designated by the inventors as 'clone B6' are made available. Said clone and, thus, the antibody and fragment thereof according to this embodiment, specifically and selectively bind to gp100. The antibody or fragment thereof according to this embodiment can be readily produced in a known manner.

Yet, another embodiment of the invention provides the antibody or fragment thereof which comprises
- the heavy chain variable domains
   - CDR3 region having an amino acid sequence being at least 90% identical to said in SEQ ID NO: 35 and/or
   - CDR2 region having an amino acid sequence being at least 90% identical to said in SEQ ID NO: 34 and/or
   - CDR1 region having an amino acid sequence being at least 90% identical to said in SEQ ID NO: 33 and/or
- the light chain variable domains
   - CDR3 region having an amino acid sequence being at least 90% identical to said in SEQ ID NO: 38 and/or
   - CDR2 region having an amino acid sequence being at least 90% identical to said in SEQ ID NO: 37 and/or
   - CDR1 region having an amino acid sequence being at least 90% identical to said in SEQ ID NO: 36.

With this measure, the antigen-binding sites responsible for antigen binding and therapeutic efficacy of the particular affine and gp100-specific antibody designated by the inventors as 'clone L1C' are made available. Said clone and, thus, the antibody and fragment thereof according to this embodiment, specifically and selectively bind to gp100. The antibody or fragment thereof according to this embodiment can be readily produced in a known manner.

In another embodiment of the invention said antibody or fragment thereof comprises
- a heavy chain having an amino acid sequence being at least 90% identical to said in SEQ ID NO: 11, and/or
- a light chain having an amino acid sequence being at least 90% identical to said in SEQ ID NO: 16.

In this embodiment, the entire amino acid sequences of the hypervariable regions of the heavy (IgH) and light chains (IgK) of the antibody of the invention (clone A7) are provided, i.e., not only the sequences of CDR1, 2, and 3, but also the sequences of the intervening 'framework regions' (FRs) FR1, FR2, FR3, and FR4. The production is thus considerably simplified, and the resulting antibody and fragment are characterized by particular suitability according to the invention for the prophylaxis and treatment of cancer and in particular melanoma.

In another embodiment of the invention the antibody or fragment thereof comprises
- a heavy chain having an amino acid sequence being at least 90% identical to said in SEQ ID NO: 27, and/or
- a light chain having an amino acid sequence being at least 90% identical to said in SEQ ID NO: 32.

In this embodiment, the entire amino acid sequences of the hypervariable regions of the heavy (IgH) and light chains (IgK) of the antibody of the invention (clone B6) are provided, i.e., not only the sequences of CDR1, 2, and 3, but also the sequences of the intervening 'framework regions' (FRs) FR1, FR2, FR3, and FR4. The production is thus considerably simplified, and the resulting antibody and fragment are characterized by particular suitability according to the invention for the prophylaxis and treatment of cancer and in particular melanoma.

In another embodiment of the invention the antibody or fragment thereof comprising
- a heavy chain having an amino acid sequence being at least 90% identical to said in SEQ ID NO: 43, and/or
- a light chain having an amino acid sequence being at least 90% identical to said in SEQ ID NO: 48.

In this embodiment, the entire amino acid sequences of the hypervariable regions of the heavy (IgH) and light chains (IgK) of the antibody of the invention (clone L1C) are provided, i.e., not only the sequences of CDR1, 2, and 3, but also the sequences of the intervening 'framework regions' (FRs) FR1, FR2, FR3, and FR4. The production is thus considerably simplified, and the resulting antibody and fragment are characterized by particular suitability according to the invention for the prophylaxis and treatment of cancer and in particular melanoma.

Another subject-matter of the invention relates to an antibody or fragment thereof configured for a specific binding to glycoprotein 100 (gp100) for use in the prophylaxis and/or treatment of cancer, preferably gp100-expressing tumors, highly preferably melanoma.

The features, characteristics, advantages and embodiments mentioned further above apply *mutatis mutandis* also for this subject-matter.

As used herein, "prophylaxis" describes the totality of all measures taken for this purpose to prevent impairment of health by risk factors, diseases or accidents. The prevention of secondary diseases or maldevelopments by timely treatment of a primary disease is also a form of prophylaxis. In relation to the invention is thought, in particular, to the prevention of the development of cancer, especially melanoma.

Prophylaxis of cancer also includes "prevention of metastasis" or "prevention of secondary tumors", i.e., measures aiming to prevent the transmission of cancerous cells from the primary tumor to one or more sites elsewhere in a patient where then secondary tumors develop. This means that the metastasis of the primary, tumor or cancer is prevented, delayed, or reduced and thus the development of secondary tumors is prevented, delayed, or reduced. Preferably the metastasis, i.e., secondary tumors, of the lung are prevented or reduced, which means that metastatic transmission of cancerous cells from the primary tumor to the lung is prevented or reduced.

According to the invention "treatment", as used in this context, refers to therapeutic measures aimed either at eliminating the cause of the disease (causal therapy) or at eliminating the symptoms (symptomatic therapy). The invention, in particular, refers to the treatment of cancer, especially melanoma. Also included is the use of the antibody or fragment thereof according to the invention for use in adjuvant therapy. Adjuvant therapy generally refers to supplementary or supportive therapeutic measures, but in particular after surgical removal of the tumor, e.g., melanoma.

"gp100-expressing tumors" are not only melanomas, but also other tumors expressing gp100. Such other tumors include, for example, melanocytic tumors, clear cell sarcomas (soft tissue melanoma-like tumors), pigmented schwannomas, and pigmented epithelial tumors. The antibody and fragments thereof according to the invention can also be successfully used for the prophylaxis and treatment of such tumors.

In another embodiment of the invention the antibody or fragment thereof is conjugated to a drug (antibody drug conjugate, ADC).

This measure further develops the antibody and fragment thereof according to the invention into a very potent therapeutic agent. An Antibody Drug Conjugate (ADC) is a targeted cancer therapeutics composed of antibody or fragment and cytotoxic payload (drug). The drug can be a substance toxic to cancer cells. Both components are typically connected by a linker, e.g. a stable chemical linker that connects the antibody to the drug and releases the payload selectively within the tumor cells. ADCs combine the specificity of antibodies with the powerful tumor-killing capabilities of cytotoxic agents, minimizing off-target effects and improving therapeutic outcomes. This embodiment allows for the selective targeting and destruction of gp100-expressing tumor cells, particularly melanoma.

The inventors were able to test monomethyl auristatin E (MMAE) -conjugated gp100-specific antibodies in an ADC assay. These gp100-ADC molecules effectively killed melanoma cells, confirming their therapeutic potential.

Toxins suitable for ADC according to the invention are well known to the skilled person. They include, e.g., not only MMAE but also monomethyl auristatin F (MMAF), maytansinoids (DM1, DM4), pyrrolobenzodiazepines (PBDs), calicheamicins, duocarmycins, topoisomerase I Inhibitors (SN-38, deruxtecan (Dxd)) etc.

Still another subject-matter of the invention relates to a pharmaceutical composition characterized in comprising an antibody of fragment thereof according to the invention.

The features, characteristics, advantages and embodiments mentioned in relation with the antibody or fragment thereof apply *mutatis mutandis* also for the pharmaceutical composition.

The pharmaceutical composition may comprise a pharmaceutical carrier. As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A pharmaceutical composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

These pharmaceutical compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Actual dosage levels of the active ingredient, i.e., antibody or fragment thereof, in the pharmaceutical composition of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

In an embodiment the antibody and/or fragment thereof are the only active ingredients of the pharmaceutical composition. In another embodiment, the pharmaceutical composition may contain additional active agents, such as anti-cancer or anti-melanoma compounds, e.g., traditional chemotherapeutics. In a preferred embodiment the pharmaceutical composition additionally comprises an immune checkpoint inhibitor (ICI).

This measure extends the invention to include combination therapy with an ICI. The clinical application is herewith broadened. By adding ICls, the therapy can address multiple resistance mechanisms in melanoma. This approach makes use of a synergistic tumor killing. The antibody or fragment thereof directly kills tumor cells, reducing tumor burden and releasing tumor antigens, while ICls enhance T-cell function, enabling the immune system to attack remaining tumor cells. This two-pronged approach increases overall treatment efficacy, may overcome immune resistance and has the potential for lower toxicity.

Potentially suitable ICIs fall into several categories: PD-1 inhibitors (pembrolizumab, nivolumab), which block the PD-1 receptor on T cells to prevent exhaustion and enhance the immune response against tumor cells, as well as PD-L1 inhibitors (atezolizumab, durvalumab), which inhibit tumor-expressed PD-L1, thereby preventing T-cell suppression. Another important group consists of CTLA-4 inhibitors (ipilimumab), which enhance early T-cell activation, leading to a stronger anti-tumor immune response. Additionally, LAG-3 inhibitors (relatlimab) help reinvigorate exhausted T cells, while experimental TIM-3 inhibitors (TSR-022) and TIGIT inhibitors (tiragolumab) offer new strategies to overcome tumor-induced immunosuppression.

A still further subject-matter according to the invention relates to a kit comprising:
a) a container comprising the antibody or fragment thereof according to the invention in solution or in lyophilized formulation;
b) optionally, a second container containing a diluent or reconstituting solution for the lyophilized formulation;
c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments mentioned in relation to the antibody or fragment thereof apply *mutatis mutandis* also to the kit.

Still another subject-matter of the invention relates to a nucleic acid molecule encoding an antibody or fragment thereof or a polypeptide according to the invention.

This measure allows the recombinant production of the antibody or fragment thereof or polypeptide, according to the invention, in an advantageous manner. Methods for recombinant production are widely known in the state of the art as has been described further above.

The features, characteristics, advantages and embodiments mentioned in relation to the antibody or fragment thereof apply *mutatis mutandis* also for the nucleic acid.

The terms "nucleic acid" or "nucleic acid molecule", as used herein interchangeably, are intended to include DNA molecules and RNA molecules. A nucleic acid molecule may be single-stranded or double-stranded, but preferably is double-stranded DNA. It includes oligonucleotide molecules.

Yet, another subject-matter of the present invention is a vector, preferably an expression vector, comprising the nucleic acid or oligonucleotide according to the invention, and, optionally, regulatory elements necessary for expression in a prokaryotic and/or eukaryotic cell.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product. "Regulatory elements" of expression vectors are well known to the skilled artisan and include, e.g., promotors, enhancers, polyadenylation signals and transcription termination sequence, etc.

The features, characteristics, advantages and embodiments mentioned in relation to the antibody or fragment thereof apply *mutatis mutandis* also for the vector.

Still another subject-matter of the invention is a prokaryotic or eukaryotic host cell comprising the vector according to the invention.

The term "host cell" as used in the current application denotes any kind of cellular system which can be engineered to generate the antibodies according to the current invention. "Host cell" herein typically refers to a cell or non-human organism genetically engineered to produce the antibodies or fragments thereof according to the invention. These hosts and host cells include mammalian cells, bacterial, yeast, insect cells, plant cells and transgenic plants or animals such as rodents, plants and bovines. Typically, antibodies or antibody fragments are expressed in mammalian, bacterial and yeast cells. In one embodiment HEK293 cells and CHO cells are used as host cells.

The features, characteristics, advantages and embodiments mentioned in relation to the antibody or fragment thereof apply *mutatis mutandis* also for the prokaryotic or eukaryotic host cell.

Another subject-matter of the invention relates to a method for the prophylaxis and/or treatment of cancer, preferably gp100-expressing tumors, highly melanoma, in a living being, comprising the administration of a prophylactically and/or therapeutically effective amount of the antibody or fragment thereof or polypeptide and/or the pharmaceutical composition and/or the nucleic acid and/or the vector according to the invention.

The features, characteristics, advantages and embodiments mentioned in relation to the antibody or fragment thereof apply *mutatis mutandis* also for the method according to the invention.

A "living being" refers to any subject or organism, including mammals, in particular humans.

The invention is now further explained by means of embodiments and examples resulting in additional features, characteristics and advantages of the invention. The embodiments and examples are of pure illustrative nature and do not limit the scope or range of the invention. The features mentioned in the specific embodiments and examples are features of the invention and may be seen as general features which are not applicable in the specific embodiment or example but also in an isolated manner in the context of any embodiment or example of the invention.

The invention is now further described and explained in more detail by referring to the following non-limiting examples and figures.

### BRIEF DESCRIPTION OF THE FIGURES

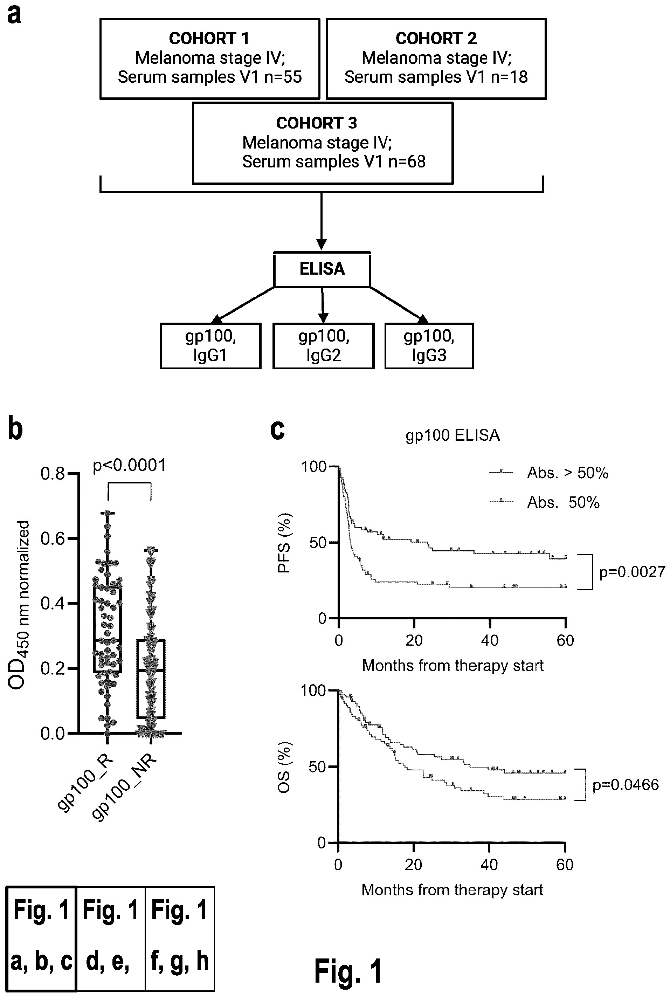

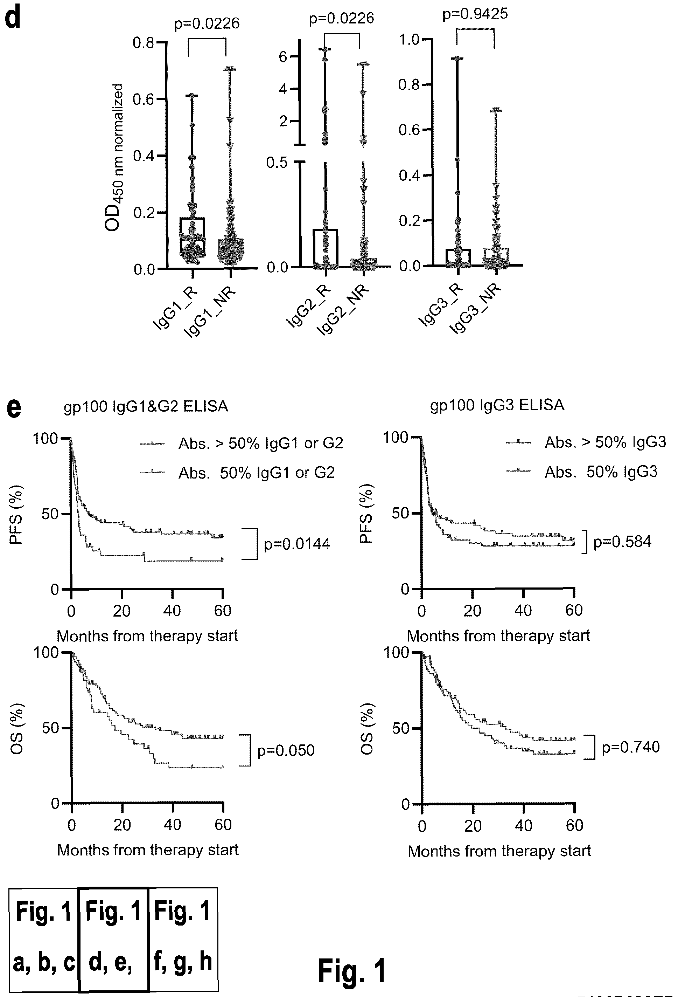

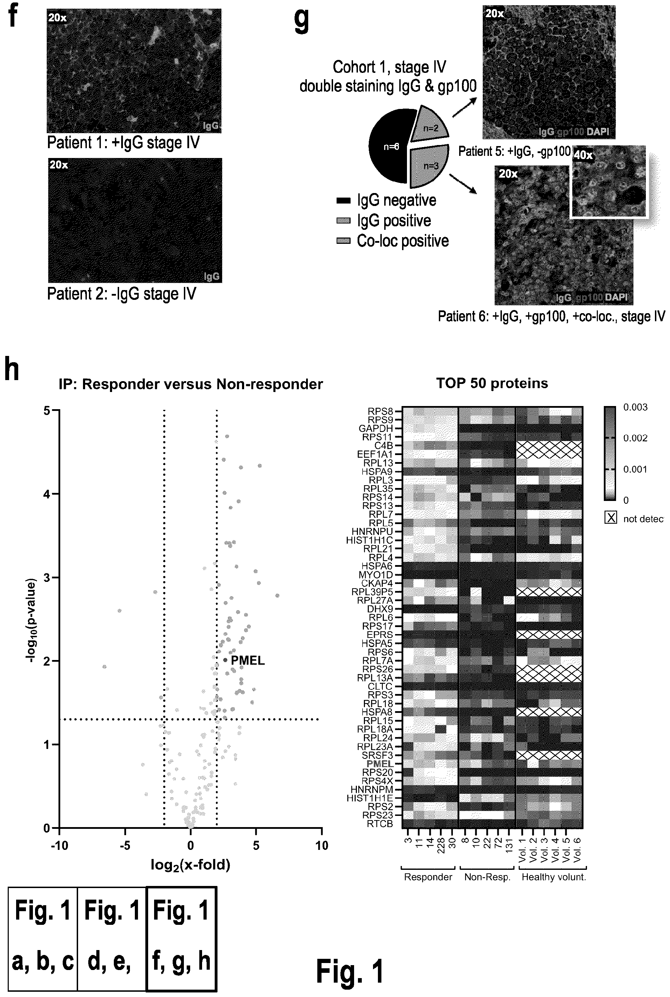

- Figure 1: Elevated antibody response against gp100 in melanoma responders to immune checkpoint inhibition.
(A) Overview of the serum samples used in three independent melanoma patient cohorts. Patients were categorized as responders (R) or non-responders (NR) to immune checkpoint inhibition (ICI) based on computed tomography (CT) scan assessments. The distribution of samples across cohorts is shown, illustrating the number of patients per response group included in the study.
(B) Gp100-specific total IgG levels in responders (n = 59) and non-responders (n = 82) measured by enzyme-linked immunosorbent assay (ELISA). Normalized optical density (OD) values at 450 nm were compared using the Mann-Whitney U test. Responders exhibited significantly elevated gp100-specific IgG levels compared to non-responders (p < 0.0001), suggesting a strong correlation between anti-gp100 IgG responses and clinical benefit from ICI.
(C) Kaplan-Meier survival analysis of progression-free survival (PFS) and overall survival (OS) in relation to gp100-specific IgG levels. Patients with gp100-IgG levels above 50% showed significantly prolonged PFS compared to those with lower levels (p = 0.0027). A similar trend was observed for OS, with patients exhibiting high gp100-IgG levels demonstrating improved survival outcomes (p = 0.0466).
(D) Gp100-specific IgG1, IgG2, and IgG3 subclass levels were measured in responders and non-responders by ELISA. Both IgG1 (p = 0.0226) and IgG2 (p = 0.0226) levels were significantly higher in responders compared to non-responders, while IgG3 levels did not differ significantly between groups (p = 0.9425), suggesting that IgG1 and IgG2, rather than IgG3, play a role in the anti-gp100 immune response.
(E) Kaplan-Meier survival analysis of progression-free survival (PFS) and overall survival (OS) stratified by IgG subclass levels. Patients with gp100-specific IgG1 or IgG2 levels above 50% exhibited significantly longer PFS (p = 0.0144) and OS (p = 0.050), whereas IgG3 levels were not associated with survival differences (p = 0.584 for PFS, p = 0.740 for OS). These findings suggest that IgG1 and IgG2 responses contribute to prolonged survival in melanoma patients receiving ICI.
(F) Immunofluorescence (IF) staining of melanoma metastases for IgG detection. Representative images depict IgG-positive and IgG-negative melanoma tissues, demonstrating heterogeneity in IgG deposition across tumor samples. IgG-positive staining was predominantly detected in the interstitial space of melanoma metastases, supporting the presence of tumor-reactive IgG in a subset of patients.
(G) Co-localization analysis of IgG and gp100 expression in melanoma tissue. Partial co-localization of IgG with gp100-positive tumor cells was observed, suggesting that gp100 is a potential antigenic target of the detected IgG response. This supports the hypothesis that endogenous anti-gp100 immune responses may contribute to melanoma tumor control in patients responding to ICI.
(H) Mass spectrometry analysis of IgG-bound proteins in melanoma patient sera. A volcano plot highlights differentially detected proteins between responders and non-responders, with gp100 (PMEL) being significantly enriched in responders. The heatmap presents the top 50 IgG-bound proteins detected across patient groups, demonstrating distinct immune reactivity patterns. Enrichment of melanoma-associated proteins, including gp100, suggests a role for gp100-specific antibody responses in mediating immune control of melanoma.

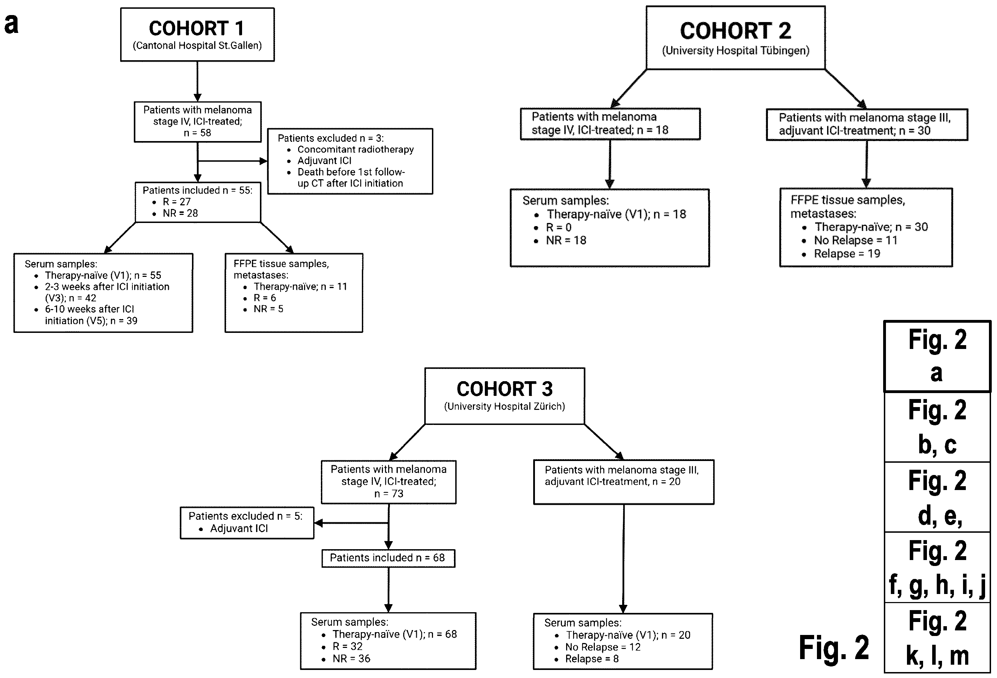

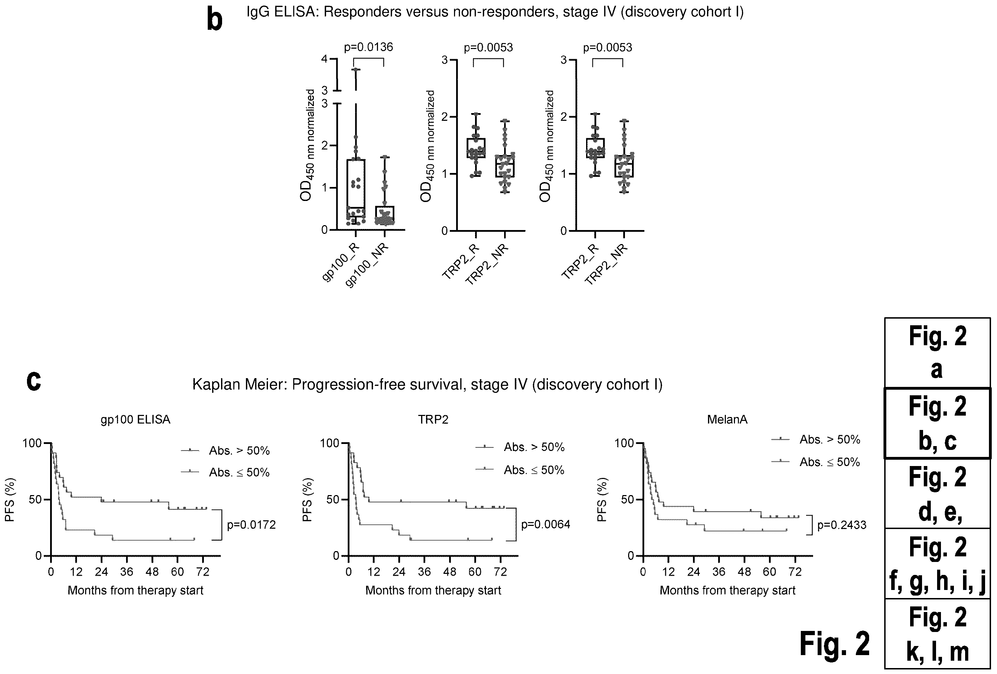

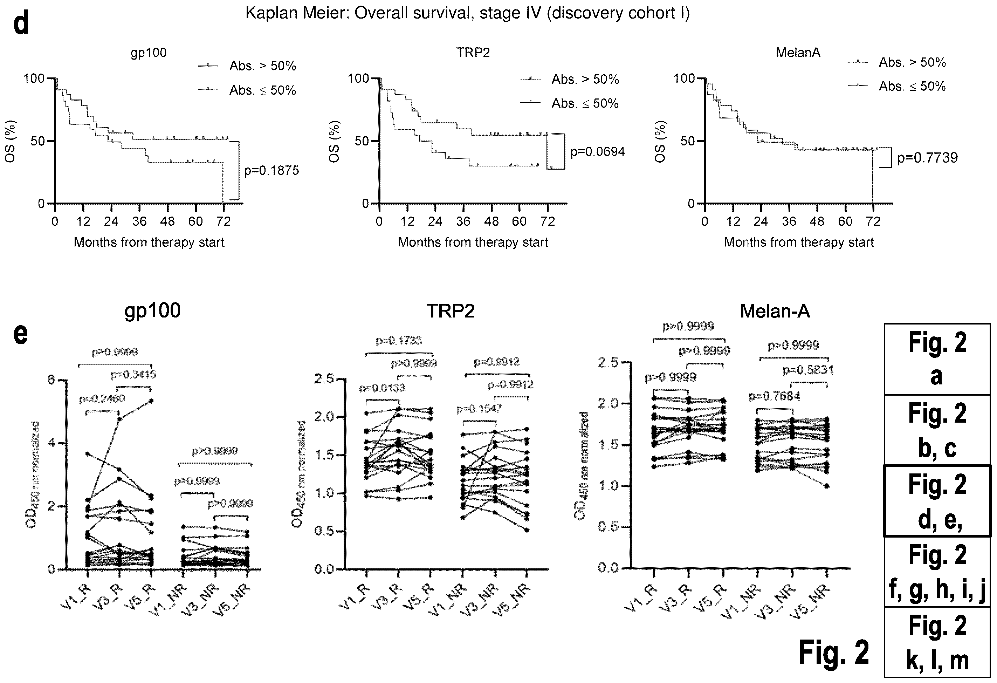

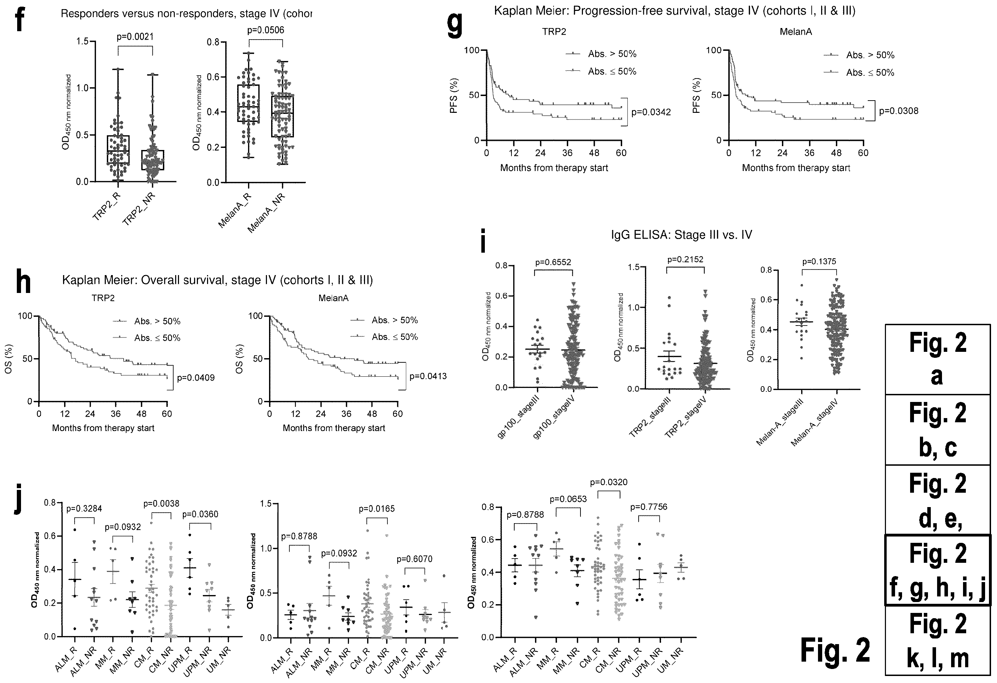

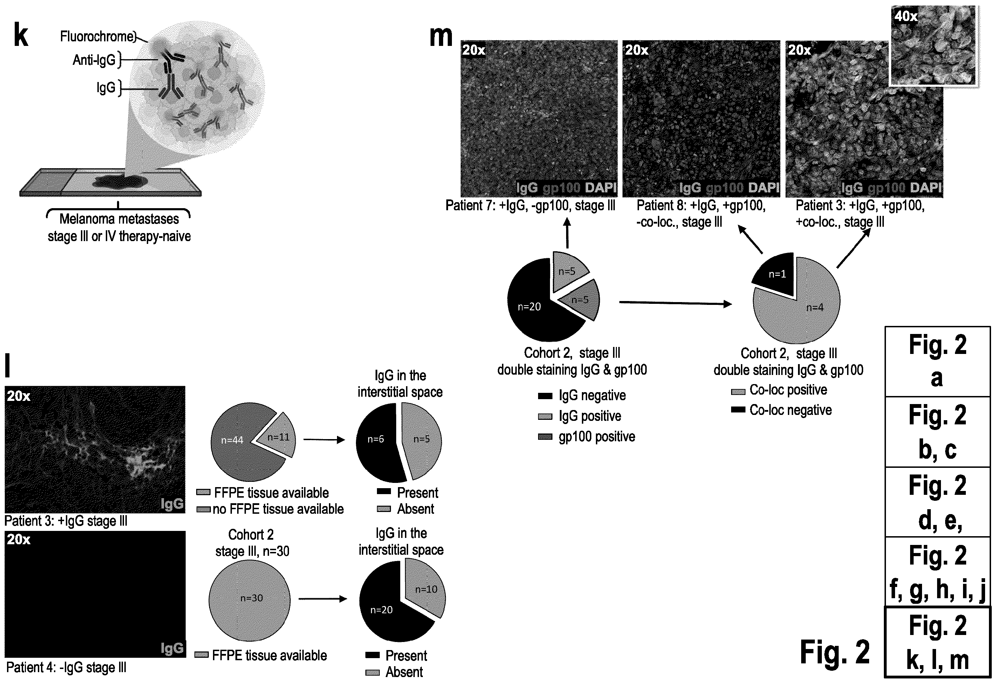

- Figure 2: Serum IgG responses in melanoma patients across cohorts and their association with clinical outcomes.
(A) Overview of the cohorts used in this study. The figure illustrates the distribution of melanoma patients across the three cohorts analyzed, highlighting their respective clinical characteristics and response classifications to immune checkpoint inhibition (ICI).
(B) Gp100-, TRP2-, and Melan-A-specific IgG levels in stage IV melanoma patients from the discovery cohort. Enzyme-linked immunosorbent assay (ELISA) was used to quantify antibody levels, with patients grouped by response status (responders, R; non-responders, NR). Differences in IgG levels between groups were assessed to evaluate the role of humoral immunity in ICI response.
(C) Kaplan-Meier analysis of progression-free survival (PFS) in stage IV melanoma patients from the discovery cohort, stratified by gp100, TRP2, and Melan-A-specific IgG positivity. Patients with high IgG levels demonstrated longer PFS compared to those with low IgG levels, supporting a potential prognostic role for antigen-specific antibody responses.
(D) Kaplan-Meier analysis of overall survival (OS) in stage IV melanoma patients from the discovery cohort, stratified by gp100, TRP2, and Melan-A-specific IgG positivity. High antibody levels were associated with prolonged OS, reinforcing the relevance of antigen-specific humoral immunity in melanoma.
(E) Longitudinal analysis of IgG titers over time in selected melanoma patients. Serial ELISA measurements of antigen-specific IgG levels revealed no major fluctuations over the course of disease progression or therapy, indicating relative stability of circulating IgG responses.
(F) Gp100-, TRP2-, and Melan-A-specific IgG levels in stage IV melanoma patients across all three analyzed cohorts. ELISA data from a combined cohort analysis confirmed trends observed in the discovery cohort, further supporting the association between humoral immunity and response to ICI.
(G) Kaplan-Meier PFS analysis across all three stage IV melanoma cohorts, stratified by gp100, TRP2, and Melan-A-specific IgG levels. Patients with high IgG titers exhibited significantly longer PFS, reinforcing the link between antigen-specific antibody responses and disease control.
(H) Kaplan-Meier OS analysis across all three stage IV melanoma cohorts, stratified by gp100, TRP2, and Melan-A-specific IgG levels. High IgG levels were consistently associated with improved OS, further supporting their prognostic potential.
(I) Comparison of gp100-, TRP2-, and Melan-A-specific IgG levels in melanoma patients with stage III (n = 20) and stage IV (n = 141) disease. Stage IV patients exhibited significantly higher antibody levels compared to stage III patients, suggesting an association between advanced disease and increased humoral responses.
(J) Comparison of antigen-specific IgG levels across different melanoma subtypes. ELISA data were analyzed to compare humoral immune responses in patients with cutaneous, mucosal, and uveal melanoma, providing insights into potential subtype-specific differences in antibody-mediated immunity.
(K) Schematic representation of the experimental workflow for detecting tumor-bound IgG in melanoma tissue using indirect immunofluorescence (IF). The approach involves incubating formalin-fixed paraffin-embedded (FFPE) melanoma sections with secondary antibodies to visualize the presence of tumor-associated IgGs.
(L) Representative example of IgG detection in a stage II melanoma metastasis using indirect IF. The image illustrates antibody deposition in the tumor microenvironment, suggesting *in situ* immune reactivity.
(M) Co-localization of gp100 and IgG in a subset of stage III melanoma samples. Immunofluorescence staining revealed partial overlap between gp100 expression and IgG deposition, supporting the hypothesis that gp100 is a target of the humoral immune response in melanoma patients.

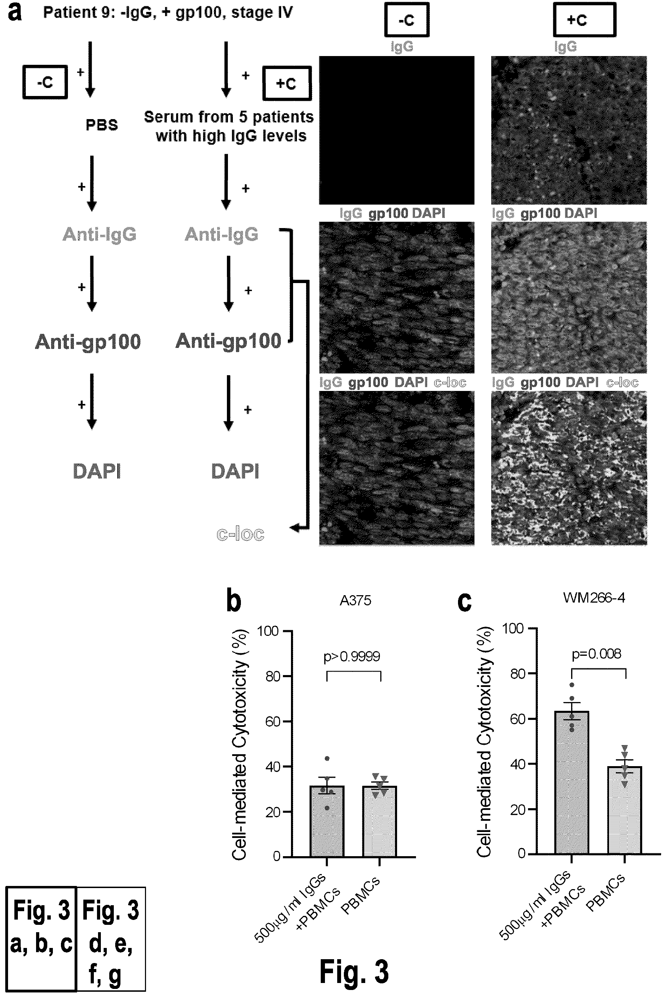

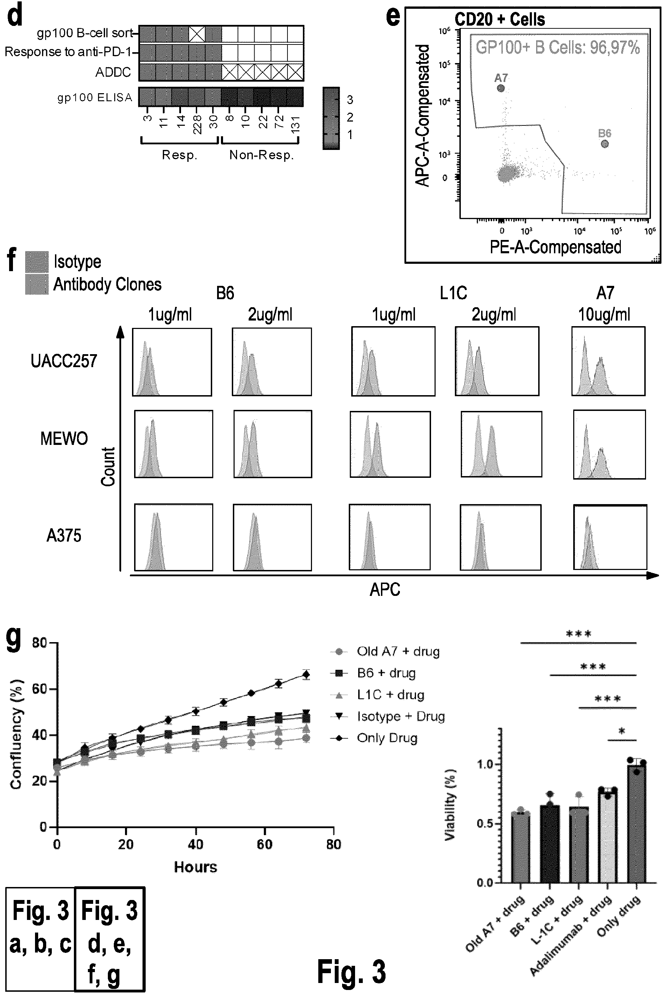

- Figure 3: Functional characterization of patient-derived gp100-specific antibodies and their cytotoxic activity.
(A) Enzyme-linked immunosorbent assay (ELISA) confirms the presence of gp100-specific IgG in serum samples from melanoma patients. To assess the antigen specificity of these IgGs of 5 patients, formalin-fixed paraffin-embedded (FFPE) sections of a previously IgG-negative tested melanoma metastasis (-C) were incubated with serum from gp100-IgG positive patients. Subsequent immunofluorescence staining revealed co-localization (+C) of patient-derived IgG with gp100-expressing tumor regions, suggesting that the detected circulating IgGs target gp100.
(B) Antibody-dependent cellular cytotoxicity (ADCC) assay using patient-derived serum. Peripheral blood mononuclear cells (PBMCs) were co-cultured with gp100-negative melanoma cells in the presence of IgGs from ELISA-positive serum samples. No significant enhancement of ADCC was observed, indicating that cytotoxic effects depend on antigen expression.
(C) ADCC assay with gp100-positive melanoma cells. When PBMCs were co-cultured with gp100-positive melanoma cells in the presence of patient-derived IgGs, a significant increase in ADCC was observed (p=0.008), suggesting that the cytotoxic potential of serum IgGs is specific to gp100-expressing melanoma cells.
(D) Schematic overview of experimental procedures performed on serum samples from five patients with high gp100-specific IgG titers. These samples were used for immunofluorescence staining, B cell isolation, and functional cytotoxicity assays to characterize the role of patient-derived antibodies in melanoma immunity.
(E) Single-cell sorting of gp100-specific B cells from peripheral blood. Fluorescence-activated cell sorting (FACS) was performed to isolate B cell receptor (BCR)-expressing memory B cells and plasmablasts specific for gp100. The sorted cells were subsequently used for sequencing and monoclonal antibody production.
(F) FACS-based binding analysis of three isolated monoclonal antibodies, A7, B6, and L1C. These clones selectively bound to gp100-positive melanoma cells but not to gp100-negative melanoma cells or non-melanoma cell lines, confirming their specificity for gp100.
(G) Antibody-drug conjugate (ADC) cytotoxicity assays demonstrating selective killing of gp100-positive melanoma cells over time. The three gp100-specific monoclonal antibodies (A7, B6, and L1C) were conjugated to monomethyl auristatin E (MMAE) and tested for cytotoxic activity. As an isotype control, an anti-TNF antibody (adalimumab) was conjugated to MMAE and used in parallel experiments. Endpoint viability assays (Alamar Blue) showed significant ADC-mediated cytotoxicity in gp100-expressing melanoma cells after 72 hours, while no significant cytotoxic effects were observed in gp100-negative cells.

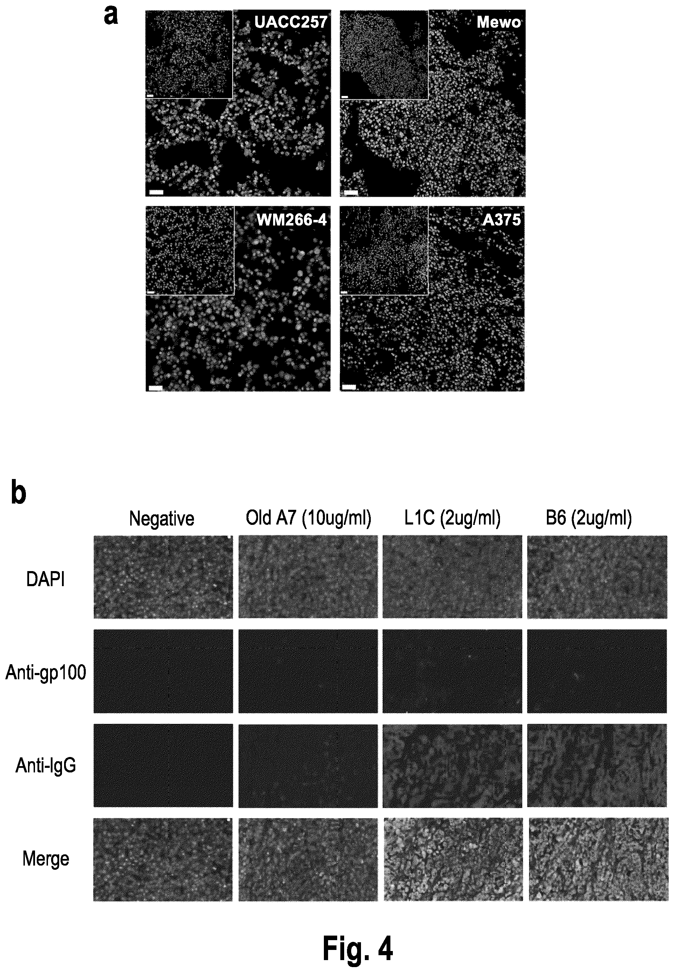

- Figure 4: Validation of gp100-specific monoclonal antibodies derived from melanoma patient B cells.
(A) Immunofluorescence (IF) staining of melanoma cell lines using gp100 specific HMB45 antibody and demonstrating differential gp100 expression. Representative images depict gp100-positive (red) and gp100-negative melanoma cell lines used for functional characterization of patient-derived antibodies. Small inlets depict the negative control stainings. Nuclei were stained DAPI (blue). The gp100-specific monoclonal antibodies were later tested on these cell lines to assess binding specificity.
   Single-cell reverse transcription polymerase chain reaction (RT-PCR) and Sanger sequencing of the heavy and light chain variable regions from the three gp100-specific monoclonal antibodies A7, B6, and L1C. The isolated B-cell receptor (BCR) sequences confirm the presence of distinct IgG clones specific for gp100.
(B) Immunofluorescence (IF) staining of gp100-positive melanoma tissue incubated with the three monoclonal antibodies A7, B6, and L1C. All three clones exhibited strong binding to gp100-positive tumor regions, confirming their specificity for gp100 in patient-derived melanoma samples.

### EXAMPLES

### 1. Methods

### Study design and clinical samples

This multicenter study entailed the prospective collection of clinical data, serum and tissue samples from the Cantonal Hospital St. Gallen, University Hospital Zürich and University Hospital Tübingen between January 2014 and March 2023. Clinical follow-up data were collected until July 2023.

Ethical approval for the study was obtained from the respective local ethics committees. The research adhered to the principles outlined in the Declaration of Helsinki and written informed consent was obtained from all participating patients.

Patients were treated either by monotherapy with an anti-PD-1 antibody (nivolumab, pembrolizumab) or anti-CTLA4 antibody (ipilimumab), or by a combination of both (nivolumab+ipilimumab).

Treatment response was assessed using RECIST version 1.1 as per investigator assessment. Tumors were staged at baseline and every eight weeks thereafter until progression or end of study treatment. Patients were categorized into responders (R), within which were sub-categories for partial response (PR) and complete response (CR); and non-responders (NR), including those with stable disease (SD) or progressive disease (PD). In stage III melanoma, relapse was defined as loco-regional or distant recurrence, or diagnosis of a new primary melanoma.

Serum samples were obtained from whole blood, aliquoted and stored at -80°C. Tissue samples of therapy-naïve melanoma metastases were collected and processed according to standard protocols for formalin-fixation and paraffin-embedding.

### ELISA

The antigens and antibodies (gp100-specific IgG, IgG1, IgG2, IgG3, IgA; TRP2-specific IgG, IgA; Melan-A-specific IgG; TRP1-specific IgG; NY-ESO-1-specifc IgG) were tested according to standard protocols. The recombinant proteins were produced in-house using HEK293 cells (see below) and were then affinity purified.

For the ELISA, 96-well flat-bottom Medisorp plates (Thermo Fisher Scientific, catalog no. 467320) or ELISA strip Medisorp plates (Thermo Fisher Scientific, catalog no. 467120) were coated with 50ng or 100ng of the recombinant protein in 100µl per well of carbonate buffer (PH 9.6) in PBS, and incubated overnight at 4°C, or for two hours at 37°C. The plates were washed with ELISA wash buffer (H₂O with 0.05%Tween-20, or PBS with 0.05%Tween-20) and blocked by adding 150µl per well of a commercial blocking agent (Monster Block (ImmunoChemistry Technologies, catalog no. 6295), or of 5% skimmed milk in PBS, or 5×ELISA/ELISPOT diluent (Thermo Fisher Scientific, catalog no. 00-4202-56), followed by incubation at room temperature (RT) for two or four hours. Sera from patients were diluted according to the protocol in a buffer (1xPBS, or 5% skimmed milk in PBS, or 10% normal goat serum (Cell signaling Technologies, catalog no. 5425S) in PBS, then 100µl of the diluted sera were added to duplicate wells and shaken for 60 minutes at RT, or for 40 minutes at 37°C and then 40 minutes at RT with shaking. After incubation and a second washing step, goat anti-human-IgG conjugated to horseradish peroxidase (Jackson ImmunoResearch, catalog no. 109-035-003), or goat anti-human-lgA conjugated to horseradish peroxidase (Jackson ImmunoResearch, catalog no. 109-035-011)/mouse anti-human IgG1 horseradish peroxidase (Southern Biotech, catalog no. 9045-05)/mouse anti-human IgG2 Fc horseradish peroxidase (Abcam, catalog no. ab99779)/mouse anti-human IgG3 Hinge-horseradish peroxidase (Southern Biotech, catalog no. 9210-05) was diluted at 1:1000 or 1:2000 in buffer (5% skimmed milk in PBS, or 10% normal goat serum in PBS), as specified in the protocol. Next, 90µl of the secondary antibody solution was added to each well and plates were shaken for 60 minutes at RT. After a third washing step, 85µl of TMB (Mabtech, catalog no. 3652-F10) were added to each well and incubated in the dark at RT for a predefined time. The reaction was stopped by adding 35µl of 2.5 N sulfuric acid (Sigma-Aldrich, catalog no. 320501-1) to each well. The light absorbance was measured by a Sunrise absorbance microplate reader (Tecan) at a wavelength of 450nm. To allow comparison of ELISA results obtained from different plates, a standard curve was included on each individual plate.

### IgG immunofluorescence

Diagnostic tissue samples taken from the metastases of 11 therapy-naïve patients with stage IV metastatic melanoma (Cantonal Hospital St. Gallen), and 30 therapy-naive patients with stage III metastatic melanoma (University Hospital Tübingen) were FFPE using standard protocols. Three-micron-thick serial sections were cut using a Leica RM2255 rotary microtome (Leica Microsystems, DE), and placed on poly-L-lysinecoated slides. The slides were dewaxed in xylene, rehydrated, subjected to heat-induced epitope retrieval (HIER) in a microwave oven using a citrate buffer (pH 6) target-retrieval solution for 20 minutes, and allowed to cool to room temperature. Non-specific antibody-binding was minimized by incubating the slides with 5% skimmed milk in PBS for 60 minutes. This was followed by an 18 hour incubation at 4°C with a Rabbit anti-Human IgG FITC antibody (Dako/Agilent, catalog no. F0202, 1:50). The next day, slides were counter-stained with DAPI and mounted using fluorescence mounting medium (Dako/Agilent, catalog no. S3023). Visualization and analysis of the stained slides were performed using an LSM980 confocal microscope with Airyscan 2 (Zeiss, DE).

### IgG and gp100 immunofluorescence

To detect IgG co-localization with gp100 in FFPE sections of melanoma metastases three-micron-thick serial sections were dewaxed in xylene, rehydrated, subjected to HIER in a microwave oven using a citrate buffer (pH 6) target retrieval solution for 20 minutes, and allowed to cool to room temperature. Once the slides reached room temperature they were blocked for 60 minutes with 5% skimmed milk in PBS. This was followed by an overnight (18 h) incubation at 4°C with a Rabbit anti-Human IgG FITC antibody (Dako/Agilent, catalog No. F0202, 1:50), and another overnight incubation at 4°C with a monoclonal Mouse anti-Human melanosome antibody (Dako/Agilent, catalog no. M063401-2, clone HMB45, 1:100). Slides were then incubated with a Donkey anti-Mouse A594 secondary antibody (Jackson Immunoresearch, catalog number 715-585-150, 1:200) for 60 minutes at RT, followed by counterstaining with DAPI, and mounting using fluorescence mounting medium (Dako/Agilent, catalog no. S3023).

To assess the binding of IgG from patients' serum to gp100 within melanoma metastases, two serial sections from an IgGneg/gp100pos stage IV melanoma metastasis were dewaxed in xylene, rehydrated, subjected to HIER in a microwave oven using a citrate buffer (pH 6) target retrieval solution for 20 minutes, and allowed to cool to room temperature. The slides were then incubated for 60 minutes with 5% skimmed milk in PBS, then for 18 hours at 4°C with either PBS, or with pooled serum from five patients who showed high IgG levels (as measured by ELISA). This was followed by 18 hours incubation at 4°C with the same Rabbit anti-Human IgG FITC antibody (Dako/Agilent, catalog No. F0202, 1:50), and another overnight incubation at 4°C with a monoclonal Mouse anti-Human melanosome antibody (Dako/Agilent, catalog no. M063401-2, clone HMB45, 1:100). Slides were then incubated with a Donkey anti-Mouse A594 secondary antibody (Jackson Immunoresearch, catalog number 715-585-150, 1:200) for 60 minutes at RT, followed by counterstaining with DAPI, and mounting using fluorescence mounting medium (Dako/Agilent, catalog no. S3023). All slides were visualized and assessed using an LSM980 confocal microscope with Airyscan 2 (Zeiss, DE).

### Recombinant protein production in HEK293 cells

The recombinant proteins gp100, TRP2, Melan-A, TRP1 and NY-ESO-1 were produced in-house. HEK293 cells were transduced with lentiviral particles or transfected with plasmids carrying the corresponding genes. Overexpression was assayed by Western Blot and recombinant proteins were purified by affinity purification. Dodecylsulfate polyacrylamide gel electrophoresis (SDS-PAGE) followed by staining with Coomassie Blue was used to determine the protein concentrations.

### Immunoprecipitation and mass spectrometry

Lysates from gp100-positive melanoma cells (UACC257) were prepared using an IP lysis buffer (25 mM Tris-HCl pH 7.4, 150 mM NaCl, 1% NP-40, 0.5% CHAPS, 1 mM EDTA, 5% glycerol) supplemented with protease inhibitors (cOmplete mini, Roche). For each experiment, 500 µg of protein lysate was used. Serum samples (50 µl) from five ICl-responsive stage IV melanoma patients, five non-responding (NR) melanoma patients, or six healthy donors were added to the lysates in a final volume of 500 µl. The mixtures were incubated overnight at 4°C under constant rotation. Subsequently, 50 µl protein G agarose beads (ROTI^{®}Garose-Protein G HPBeads, Roth) were added to the mixtures and incubated at 4°C for 2 hours with rotation. After five washes with PBS containing 0.05% Tween-20, the bound protein complexes were eluted in 80 µl 2x Lämmli buffer. Proteins were resolved via short-term SDS-PAGE and stained with Coomassie Brilliant Blue R-250 (1610436, Bio-Rad Laboratories).

For protein identification, excised protein bands were subjected to in-gel tryptic digestion. Extracted peptides were desalted using C18 StageTips and analyzed by liquid chromatography-tandem mass spectrometry (LC-MS/MS). LC-MS/MS was performed on an Easy-nLC 1200 UHPLC (Thermo Fisher Scientific) coupled to an Orbitrap Exploris 480 mass spectrometer (Thermo Fisher Scientific). Peptides were eluted using a 60-minute gradient at a flow rate of 200 nL/min, with the 20 most intense precursor ions selected for fragmentation by higher-energy C-trap dissociation. Mass spectrometry data were processed using MaxQuant software suite (v.1.6.7.047) for protein identification and quantification. Intensity-based absolute quantification (iBAQ) values were calculated and normalized to the IgG heavy chain signal. Enrichment of proteins in ICl-responsive patients was statistically compared to non-responding patients and healthy donors using x-fold enrichment and t-tests. Candidate proteins showing significant enrichment in ICI responders were further analyzed.

### Cell lines

UACC257, Sk-Mel-5, WM266-4 and A375 were purchased. Cells were cultured in standard medium with a renewal of fresh medium every other day. These cells were used for mass spectrometry or lactate dehydrogenase assay or ADC assay.

### Lactate dehydrogenase (LDH) cytotoxicity assay

ADCC was determined by released LDH activity assay using the melanoma cell-lines WM266-4 and A375 and a Cytotoxicity Detection Kit (Roche, catalog no.11644793001). LDH release was tested in five replicate wells per condition, in the following combinations: cancer cells+IgG+PBMCs, cancer cells+PBMCs, cancer cells+medium, cancer cells+1% Triton X-100 (Sigma-Aldrich, catalog no. T8787), PBMCs+medium, and medium only. Prior to assessment, WM266-4 and A375 cells were transferred to 96-well flat-bottom tissue culture plates (TPP, catalog no. 92696) and incubated overnight at 37°C. Where IgG were included, they were diluted to 500µg/ml then added and incubated for 30 minutes at 37°C. Fresh PBMCs from a healthy donor were isolated using Ficoll density gradient separation and added at a ratio of 40:1 to the cancer cells, followed by incubation for 20 hours at 37°C. The activity of released LDH was measured by absorption of the chromogenic substrate at 490nm. After subtracting the background level of absorbance from each test value, the percentage of cell-mediated cytotoxicity was calculated using the formula: ((Experimental value - PBMCs spontaneous release control) - low control))/(high control - low control) x100.

The IgGs used in the LDH cytotoxicity assay were purified and concentrated. Serum purification was accomplished using the Melon Gel IgG Spin Purification Kit (Thermo Fisher Scientific, catalog no. 45206), following the manufacturer's protocol. After purification, IgG was concentrated utilizing an Amicon Ultra-15 Centrifugal Filter Device (Merck Millipore, catalog no. UFC901024). The concentration of IgGs was quantified using NanoDrop One (Thermo Fisher Scientific).

### FACS Sorting and Staining

A total of 8 × 10⁷ pooled PBMCs from four ICl-responder patients were magnetically enriched using the Memory B Cell Isolation Kit (130-093-546, Miltenyi). B cells were further enriched by FACS sorting of CD20⁺ antigen-binding small lymphocytes using the Sony MA900 cell sorter. Additionally, 2 × 10⁶ pooled cells from the lymph nodes of two patients were used directly for FACS sorting. Brilliant Violet 421-conjugated anti-IgG (410704, BioLegend) and Brilliant Violet 605-conjugated anti-CD19 (302244, BioLegend) were used.

Pooled cells were treated with Benzonase Nuclease (70664, Sigma-Aldrich) at 37°C for one hour to reduce cell clumping. Cells were then stained with antibodies and biotinylated gp100 at 4°C for 30 minutes. After washing, cells were incubated with APC- or PE-conjugated streptavidin (405207 & 405203, BioLegend) at 4°C for 30 minutes before sorting. Single cells were sorted into a 96-well PCR plate containing 4 µL of lysis buffer per well, following a standard protocol.

Avi-tagged gp100 was recombinantly expressed in A375 melanoma cells and biotinylated using BirA ligase (BI001, GeneCopoeia) before staining with APC- or PE-conjugated streptavidin.

For flow cytometry staining, cells were detached from T75 flasks using 10 mM EDTA, incubated with Zombie Aqua^{™} viability dye (423108, BioLegend) and Fc Block (422302, BioLegend) at room temperature for 20 minutes. The cells were then stained with various antibody clones at different concentrations at 4°C for 30 minutes. After washing, cells were incubated with APC-conjugated anti-human Fc antibody (410712, BioLegend) at 4°C for 30 minutes. The samples were then prepared for flow cytometry analysis.

### RT-PCR, Nested PCR, and Sanger Sequencing

RT-PCR and nested PCR were performed as described before. Briefly, SuperScript^{™} IV Reverse Transcriptase (Thermo Fisher, #18090050) and random primers (Thermo Fisher, #48190011) were used for the initial RT-PCR. Subsequently, GoTaq^{®} G2 Hot Start Taq Polymerase (Promega, #M7406) was employed for nested PCR. For DNA electrophoresis, 10 µL of each PCR product was loaded onto a 0.8% agarose gel (Sigma-Aldrich, #A9539) and separated by electrophoresis. Correct bands were excised and purified using the QIAquick Gel Extraction Kit (QIAGEN, #28506). The purified DNA was submitted to Microsynth for Sanger sequencing.

### ADC Assay

A total of 4,000 cells per well were seeded in 50 µL of medium in a 96-well plate with CellTox^{™} Green (G8742, Promega) following the kit protocol. The cells were cultured overnight to allow adherence. Antibodies and drugs were conjugated using the Antibody-Drug Conjugation Kit (AT7001-500, AlphaThera). Briefly, 2 µg of each antibody clone was diluted in 30 µL PBS, and 2 µL of the dissolved drug was added to the antibody solution. The mixture was then exposed to UV light at 366 nm for two hours to facilitate conjugation. The resulting antibody-drug conjugates (ADCs) were diluted to various concentrations in RPMI medium, and 50 µL of each dilution was added to the respective wells. The cells were then cultured for 72 hours, with images captured every two hours.

A total of 4,000 cells per well were seeded in 50 µL of medium in a 96-well plate with CellTox^{™} Green (G8742, Promega) following the kit protocol. The cells were cultured overnight to allow adherence. Antibodies and drugs were conjugated using the Antibody-Drug Conjugation Kit (AT7001-500, AlphaThera). Briefly, 2 µg of each antibody clone was diluted in 30 µL PBS, and 2 µL of the dissolved drug was added to the antibody solution. The mixture was then exposed to UV light at 366 nm for two hours to facilitate conjugation. The resulting antibody-drug conjugates (ADCs) were diluted to various concentrations in RPMI medium, and 50 µL of each dilution was added to the respective wells. The cells were then cultured for 72 hours, with images captured every two hours.

### Alamar blue assay

The Alamar Blue assay was used to assess the cytotoxic effects of antibody-drug conjugates (ADCs) on gp100-positive melanoma cells. Briefly, melanoma cells were seeded in 96-well plates and treated with ADCs for 72 hours. Alamar Blue stock solution (1 mg/ml) was pre-diluted in culture medium (1:10), and 10 µl of this solution was added to 100 µl of culture medium per well. After incubation for 1 hour at 37 °C, fluorescence intensity was measured using a microplate reader (Berthold, Germany) at excitation/emission wavelengths of 540 nm/640 nm. Background-subtracted fluorescence values were used to determine cell viability, with untreated cells serving as a reference control.

### Statistical analysis

GraphPad Prism version 8.4.3 was used for statistical analysis, except for determining the optimal ELISA absorbance cutoff points, where R 4.2.0 software was used. The optimal cutoff points were defined by the "maxstat" package (version 0.7-25) using the maximally selected log-rank statistics and specifying a minimum proportion of 30% per group [33]. Kaplan Meyer curves were used to visualize and compare the PFS/OS between groups whose levels of IgG were above or below the optimal cutoff point, using log-rank test. PFS/OS was calculated from the first dose of ICI until progression of disease or death, or of censoring of the data. The association between ELISA absorbance values and therapy response/melanoma stage at diagnosis was assessed using the Mann-Whitney test. To compare ELISA values among subgroups of melanoma, the Kruskal-Wallis test was used, followed by Dunn's multiple comparisons test. For evaluating variations in ELISA values over time, the Friedmann test was applied. The difference in the LDH release between PBMCs alone and IgGs+PBMCs was examined by the Mann-Whitney test.

All illustrations were created with BioRender.com

### 2. Results

The inventors collected clinical data, tissue, and serum samples from patients being treated at three institutions (Figure 1a). In total, they analyzed samples from 149 patients with stage IV melanoma that were initiating ICI treatment, with data from 141 of these patients included in the final analysis. Of these, 59 (41.8%) were responders (R) and 82 (58.2%) non-responders (NR). Median follow-up for stage IV patients was 57 months, with median progression-free survival (PFS) of five and median overall survival (OS) of 25 months. Alongside, the inventors studied samples from 50 patients with stage III melanoma who were receiving adjuvant ICI. They obtained tissue and serum samples from 30 and 20 of these patients, respectively. Within this stage III cohort, 27 patients (54%) experienced a relapse. Here, the median recurrence-free survival (RFS) was 11 months, while the median overall survival (OS) has not been reached yet.

The inventors first used enzyme-linked immunosorbent assays (ELISAs) to investigate the presence of IgG targeting gp100, TRP2, Melan-A, TRP1 or NY-ESO-1 in the serum of 55 patients with stage IV melanoma (the discovery cohort or cohort 1). They collected blood from patients prior to the start of treatment at the first visit (V1, n = 45) and, if possible, at the third visit in weeks 2-3 (V3, n = 42) and at the fifth visit in weeks 6-10 (V5, n = 39) after ICI initiation (Figure 2a). They observed significantly higher baseline anti-gp100 and anti-TRP2 IgG levels in patients that went on to become ICI responders (n = 21), compared to non-responders (n = 24) (Figure 2b). The inventors noted a significantly prolonged PFS in patients exhibiting elevated pre-treatment IgG levels (above the median) targeting gp100 and TRP2, as well as by trend Melan-A (Figure 2c). Elevated pre-treatment levels of these MDA-specific IgG were also by trend correlated with a higher likelihood of prolonged OS for IgG against gp100 and TRP2 (Figure 2d).

The inventors next asked whether these notable correlations between MDA-specific IgG levels and therapy response/survival were associated with changing antibody levels during treatment. Interestingly, we did not detect any significant change in IgG levels targeting gp100, TRP2 or Melan-A between the sampling time points in either R or NR groups (Figure 2e).

The inventors expanded their ELISA results in a multicenter cohort (I, II & III) analyzing baseline gp100-specific IgG. Additionally, they analyzed levels of IgG1, IgG2, and IgG3 targeting gp100 before therapy. ELISAs were performed with serum samples from all three cohorts including 141 patients with melanoma stage IV plus 20 patients with stage III melanoma. In patients with stage IV melanoma, the inventors observed significantly higher gp-100 specific IgG levels in ICI responsive (R; n = 59) compared to non-responsive patients (NR; n = 82) (Figure 1b). Similar results were obtained for anti-TRP2 IgG, alongside a supporting trend for anti-Melan-A IgG (Figure 2f). Elevated IgG levels, meaning ELISA absorption values above the median, with specificity to gp100 correlated with an increased probability of an extended PFS and for gp100. as well as TRP2 and Melan-A again OS (Figure 1c, 2g & h). Baseline IgG levels in patients with melanoma stage IV (n = 141) and stage III (n = 20), were comparable (Figure 2i).

The inventors further analyzed gp-100-, TRP2-, and Melan-A-specific IgG antibodies across melanoma variants, categorized by clinical and pathological traits: UV-related cutaneous melanoma (superficial spreading melanoma + nodular melanoma) (n = 89, R = 43, NR = 46), acral lentiginous melanoma (n = 17, R = 5, NR = 12), mucosal melanoma (n = 13, R = 5, NR = 8), uveal melanoma (n = 5, R = 0, NR = 5) and melanoma of unknown primary (n = 15, R = 6, NR = 9) with two exclusions for indeterminate subtype. No significant difference was observed in gp-100-, TRP2- and Melan-A-specific IgG levels between the different subtypes (Figure 2j). In a subgroup analysis, significant differences in R versus NR were noted in gp100-, IgGs in patients with cutaneous melanoma and with melanoma of unknown primary (Figure 2j). Since elevated gp100-specific IgGs showed the most pronounced correlation with response and survival in the context of ICI, we focused on the role of IgG1, IgG2, and IgG3 targeting gp100. The inventors observed that significantly elevated IgG1 or IgG2 levels targeting gp100 are associated with response (Figure 2d) and a longer PFS and OS (Figure 2e), whereas IgG3 signals remained very low and did not correlate with survival (again, samples were grouped according to their absorption values in the ELISA into > 50% or ≤ 50%).

The next aim of the inventors was to evaluate the presence of IgG in relation to gp100 expression in melanoma metastases as the mere presence in serum does not necessarily indicate the presence of immunoglobulins in the melanoma microenvironment. First, they asked how many of these tumors contained IgG. Immunofluorescence on FFPE metastasis samples from therapy-naïve patients with stage IV melanoma showed that in five out of 11 (45%), IgG was present within the tumor tissue (Figures 2k, 1f). Similarly, 10 out of 30 (33%) metastasis samples from therapy-naïve patients with stage III melanoma contained IgG (Figure 2l). Next, the inventors performed double immunofluorescence for IgG and gp100 on the IgG-positive tumors. In cohort one, three out of five (60%) IgG-positive metastases were also gp100-positive and co-localization was detected (Figure 1g). In cohort II, five out of 10 (50%) IgG-positive metastases exhibited a signal for gp100; co-localization was observed in four of these metastases (80%) (Figure 1m).

To confirm and extend the co-localization image data, the inventors asked directly whether serum from patients could bind gp100 from cancer cells. They selected five ICI responsive patients from cohort one with stage IV melanoma and upper quartile levels of IgG recognizing gp100, TRP2, and Melan-A measured by ELISA, and performed immunoprecipitations using their serum and the lysate of gp100⁺ melanoma cells (UACC257) followed by LS-MS/MS for protein identification. The inventors identified antigen candidates that were significantly enriched in the ICI responders, including GAPDH, ribosomal proteins (RPS8, RPS9 and RPL13), and gp100 (Figure 1h). The comparator group with serum from five NR patients showed significantly less binding to gp100 in the mass spec data, as did sera from a group of six healthy donors (Figure 1h).

The results suggest that pre-existing MDA and gp100-specific IgG1s and IgG2s serve as suitable biomarkers for response and PFS to ICI, and also have the ability to directly bind gp100 expressed by tumor cells *in vivo* and *in vitro.* The inventors, therefore, next investigated the potential for these antibodies to directly mediate a humoral anti-melanoma response. The prerequisite for such an effect on a solid tumor is the binding of patient IgGs to gp100⁺ melanoma cells in a melanoma metastasis. The inventors, therefore, inoculated a FFPE section from an IgG-negative but gp100-positive metastasis with pooled serum from the previously assayed five ICl-responders. In contrast to the original IgG-negative sample, a co-localization between IgG and gp100 was detected after staining with the pooled IgG from these patients (Figure 3a). The inventors then asked whether serum samples containing MDA-specific IgGs from patients could not only bind to but also induce the killing of melanoma cells in vitro via antibody-dependent cell-mediated cytotoxicity (ADCC). IgGs isolated and concentrated from serum samples of responders were incubated with either gp100⁺ WM266-4 or gp100⁻ A375 melanoma cells, in the presence of peripheral blood mononuclear cells (PBMCs) from a healthy donor. gp100 expression was verified using immunofluorescence (Figure 4a) and cell killing was measured by lactate dehydrogenase (LDH) cytotoxicity assay. The inventors observed a significant increase in cell-mediated cytotoxicity when IgGs and PBMCs were applied to gp100-positive WM266-4 cells, compared to when PBMCs alone were added, indicative of an antibody-dependent effect. By contrast, antibody-independent LDH release was comparable in gp100-negative A375 cells incubated with PBMCs alone or in combination with patient-derived IgGs (Figure 3b,c). Next, the inventors used pooled PBMCs from four of the responders and performed an magnetic enrichment for switched memory B cells followed by an index sort for gp100-specific CD20⁺ B cells using mono-biotinylated gp100 (Figure 2d,e). Similarly, the inventors also used cells from two lymph nodes and performed index sorting for CD19⁺ IgG⁺ gp100-specific B cells. The isolated single cells were further processed by IgG-specific RT-PCR for the variable heavy (VH) and light chains (VL) followed by Sanger sequencing to obtain the CDRs of the B cell receptors and to clone gp100-specific, recombinant human IgG1s (Figure 2b). The recombinant IgG1 clones A7, B6 and L1C strongly bound to gp100⁺ UACC257 and MeWo cells, but bound only weakly to gp100⁻ A375 melanoma cells (Figure 3f). Then, the inventors tested the binding of the antibodies to melanoma tissue. They used FFPE samples of gp100 negative tumors for stainings with a commercial anti-gp100 (HMB45) and our antibody clones. The results showed that patient-derived antibody clones could bind to the melanoma cells, and showed partially colocalization with the commercial antibody signal (Figure 3c).

In a concluding step, the inventors employed the recombinant gp100-specific IgG1 clones A7, B6 and L1C, derived from ICl-responding patients, in ADC (antibody-drug conjugates) assays. The assays were conducted using antibody-MMAE conjugates, aiming to show ADC mediated killing of gp100⁺ UACC257 melanoma cells over three days by measuring the confluency of the cultures over time. All three drug-conjugated antibody clones inhibited UACC257 cell growth compared to the isotype group or only drug group in vitro. 72 hours after adding the conjugates, the inventors conducted an alamar blue assay to detect cell viability, which confirmed the cytotoxic effects of the three MMAE-conjugates (Figure 3g).

### 3. Overview

The inventors explored the role of MDA-specific antibodies in patients with advanced stage melanoma undergoing ICI treatment and isolated monoclonal gp100-specific antibodies from ICl-treated patients. Initially, the inventors examined the correlation between these antibodies and survival/response to ICI, finding MDA-specific antibodies potentially superior to cancer testis antigen-specific antibodies as biomarkers. The inventors demonstrated the cytotoxic effects of patients' serum through ADCC on melanoma cells *in vitro* and their ability to bind cancer cells *in vivo,* highlighting the functional significance of MDA-specific IgGs. While previous research may have understated the contribution of humoral immunity to cancer immune surveillance, the results may indicate an active involvement of MDA-specific antibodies. This assertion is supported by studies in the art that have described B cell-mediated effects on tumor-infiltrating CD8⁺ T cells. Gp100-specific IgG1 and IgG2 emerged as biomarkers for ICI response and PFS. Subsequent analysis revealed gp100-specific IgG presence in melanoma metastases, suggesting their potential therapeutic relevance.

The inventors developed recombinant monoclonal gp100-specific antibodies, demonstrating their efficacy in killing melanoma cells through ADCC and ADC *in vitro.* In summary, the inventors' data highlight the promise of MDA-specific antibodies in melanoma therapy and suggests avenues for further investigation and combination therapies.

### SEQUENCES

1. Clone A7
   Heavy chain CDR1 amino acid
      SYEMH (SEQ ID NO: 1)
   Heavy chain CDR2 amino acid
      GMSYDGSKTFHSESVKG (SEQ ID NO: 2)
   Heavy chain CDR3 amino acid
      FNYYDGSGSMPGFDL (SEQ ID NO: 3)
   Light chain CDR1 amino acid
      RASQSVGSNYLA (SEQ ID NO: 4)
   Light chain CDR2 amino acid
      GASSRAA (SEQ ID NO: 5)
   Light chain CDR3 amino acid
      HQYGRSRDYI (SEQ ID NO: 6)
   Heavy chain FR1 amino acid
      VQLVEAGGGAVQTGKSLRLSCVASGFGFS (SEQ ID NO: 7)
   Heavy chain FR2 amino acid
      WVRQAPGKGLEWVA (SEQ ID NO: 8)
   Heavy chain FR3 amino acid
      RLTISRDNSKNTLYLQMNSLRGEDTALYFCAR (SEQ ID NO: 9)
   Heavy chain FR4 amino acid
      WGQGTMVIVSS (SEQ ID NO: 10)
   Heavy chain variable region full amino acid 
VQLVEAGGGAVQTGKSLRLSCVASGFGFSSYEMHWVRQAPGKGLEWVAGMSYDGSK
TFHSESVKGRLTISRDNSKNTLYLQMNSLRGEDTALYFCARFNYYDGSGSMPGFDLWG
QGTMVIVSS (SEQ ID NO: 11)
   Light chain FR1 amino acid
      PVTLSLSPGERATLSC (SEQ ID NO: 12)
   Light chain FR2 amino acid
      WFQQKPGQAPRLLIY (SEQ ID NO: 13)
   Light chain FR3 amino acid
      GIPDRFSGSGSGTDFTLTISSLEPEDFAVYYC (SEQ ID NO: 14)
   Light chain FR4 amino acid
      FGQGTKVDIK (SEQ ID NO: 15)
   Light chain variable region full amino acid 
PVTLSLSPGERATLSCRASQSVGSNYLAWFQQKPGQAPRLLIYGASSRAAGIPDRFSGS
GSGTDFTLTISSLEPEDFAVYYCHQYGRSRDYIFGQGTKVDIK (SEQ ID NO: 16)
2. Clone B6
   Heavy chain CDR1 amino acid
      TYSMN (SEQ ID NO: 17)
   Heavy chain CDR2 amino acid
      SISDTSIYMYYVDSVQG (SEQ ID NO: 18)
   Heavy chain CDR3 amino acid
      SRPSRISAQFGMDV (SEQ ID NO: 19)
   Light chain CDR1 amino acid
      RASQSISNYLN (SEQ ID NO: 20)
   Light chain CDR2 amino acid
      AASSLHS (SEQ ID NO: 21)
   Light chain CDR3 amino acid
      QQSNSFPLT (SEQ ID NO: 22)
   Heavy chain FR1 amino acid
      VQLVESGGGLVKPGGSLRLSCVASGFTFS (SEQ ID NO: 23)
   Heavy chain FR2 amino acid
      WVRQAPGKGLEWVS (SEQ ID NO: 24)
   Heavy chain FR3 amino acid
      RFTISRDNAKNSLYLQMNSLRVDDTAVYYCAR (SEQ ID NO: 25)
   Heavy chain FR4 amino acid
      WGQGTTVTVSS (SEQ ID NO: 26)
   Heavy chain variable region full amino acid 
VQLVESGGGLVKPGGSLRLSCVASGFTFSTYSMNWVRQAPGKGLEWVSSISDTSIYMY
YVDSVQGRFTISRDNAKNSLYLQMNSLRVDDTAVYYCARSRPSRISAQFGMDVWGQGT
TVTVSS (SEQ ID NO: 27)
   Light chain FR1 amino acid
      PFSLSASVGDRVTITC (SEQ ID NO: 28)
   Light chain FR2 amino acid
      WYQREPGKAPKILIY (SEQ ID NO: 29)
   Light chain FR3 amino acid
      GVPSRFSGSGSGTEFTLTISGLQPEDSAIYFC (SEQ ID NO: 30)
   Light chain FR4 amino acid
      FGGGTKVEI (SEQ ID NO: 31)
   Light chain variable region full amino acid 
PFSLSASVGDRVTITCRASQSISNYLNWYQREPGKAPKILIYAASSLHSGVPSRFSGSGS
GTEFTLTISGLQPEDSAIYFCQQSNSFPLTFGGGTKVEI (SEQ ID NO: 32)
3. Clone L1C
   Heavy chain CDR1 amino acid
      DYDMN (SEQ ID NO: 33)
   Heavy chain CDR2 amino acid
      FIRGKAYGGTTEYAASVKG (SEQ ID NO: 34)
   Heavy chain CDR3 amino acid
      GFRRVVKHYFDSSGYFGKFDF (SEQ ID NO: 35)
   Light chain CDR1 amino acid
      RASQGIRNDLG (SEQ ID NO: 36)
   Light chain CDR2 amino acid
      AASSLQS (SEQ ID NO: 37)
   Light chain CDR3 amino acid
      LQHNNYPWT (SEQ ID NO: 38)
   Heavy chain FR1 amino acid
      EVQLVESGGGLVKPGRSLRLSCTTSGFTFG (SEQ ID NO: 39)
   Heavy chain FR2 amino acid
      WFRQAPGKGLEWVG (SEQ ID NO: 40)
   Heavy chain FR3 amino acid
      RFTISRDDSKSVAYLQMNSLKTEDTAVYYCTR (SEQ ID NO: 41)
   Heavy chain FR4 amino acid
      WGQGTLVTVSS (SEQ ID NO: 42)
   Heavy chain variable region full amino acid 
EVQLVESGGGLVKPGRSLRLSCTTSGFTFGDYDMNWFRQAPGKGLEWVGFIRGKAYG
GTTEYAASVKGRFTISRDDSKSVAYLQMNSLKTEDTAVYYCTRGFRRVVKHYFDSSGYF
GKFDFWGQGTLVTVSS (SEQ ID NO: 43)
   Light chain FR1 amino acid
      PVSLSASVGDRVTITC (SEQ ID NO: 44)
   Light chain FR2 amino acid
      WYQQKPGKAPKRLIY (SEQ ID NO: 45)
   Light chain FR3 amino acid
      GVPSRFSGSGSGTEFTLTISSLQPEDFATYYC (SEQ ID NO: 46)
   Light chain FR4 amino acid
      FGQGTKVEIK (SEQ ID NO: 47)
   Light chain variable region full amino acid

PVSLSASVGDRVTITCRASQGIRNDLGWYQQKPGKAPKRLIYAASSLQSGVPSRFSGSG
SGTEFTLTISSLQPEDFATYYCLQHNNYPWTFGQGTKVEIK (SEQ ID NO: 48)

## Claims

1. An antibody or fragment thereof specifically binding to glycoprotein 100 (gp100), **characterized in** comprising
- as heavy chain variable domain a CDR3 region, said CDR3 region has an amino acid sequence being at least 90% identical to one selected from those in SEQ ID NO: 3, 19 or 35, and/or
- as light chain variable domain a CDR3 region, said CDR3 region has an amino acid sequence being at least 90% identical to one selected from those in SEQ ID NO: 6, 22, or 38.

2. The antibody or fragment of claim 1, **characterized in that**
- the heavy chain variable domain comprises a CDR2 region, said CDR2 region has an amino acid sequence being at least 90% identical to one selected from those in SEQ ID NO: 2, 18 or 34, and/or
- the light chain variable domain comprises a CDR2 region, said CDR2 region has an amino acid sequence being at least 90% identical to one selected from those in SEQ ID NO: 5, 21 or 37.

3. The antibody or fragment of claim 1 or 2, **characterized in that**
- the heavy chain variable domain comprises a CDR1 region, said CDR1 region has an amino acid sequence being at least 90% identical to one selected from those in SEQ ID NO: 1, 17 or 33, and/or
- the light chain variable domain comprises a CDR1 region, said CDR1 region has an amino acid sequence being at least 90% identical to one selected from those in SEQ ID NO: 4, 20 or 36.

4. The antibody or fragment of any of claims 1-3, **characterized in** comprising
- the heavy chain variable domains
- CDR3 region having an amino acid sequence being at least 90% identical to said in SEQ ID NO: 3 and/or
- CDR2 region having an amino acid sequence being at least 90% identical to said in SEQ ID NO: 2 and/or
- CDR1 region having an amino acid sequence being at least 90% identical to said in SEQ ID NO: 1 and/or
- the light chain variable domains
- CDR3 region having an amino acid sequence being at least 90% identical to said in SEQ ID NO: 6 and/or
- CDR2 region having an amino acid sequence being at least 90% identical to said in SEQ ID NO: 5 and/or
- CDR1 region having an amino acid sequence being at least 90% identical to said in SEQ ID NO: 4.

5. The antibody or fragment of any of claims 1-3, **characterized in** comprising
- the heavy chain variable domains
- CDR3 region having an amino acid sequence being at least 90% identical to said in SEQ ID NO: 19 and/or
- CDR2 region having an amino acid sequence being at least 90% identical to said in SEQ ID NO: 18 and/or
- CDR1 region having an amino acid sequence being at least 90% identical to said in SEQ ID NO: 17 and/or
- the light chain variable domains
- CDR3 region having an amino acid sequence being at least 90% identical to said in SEQ ID NO: 22 and/or
- CDR2 region having an amino acid sequence being at least 90% identical to said in SEQ ID NO: 21 and/or
- CDR1 region having an amino acid sequence being at least 90% identical to said in SEQ ID NO: 20.

6. The antibody or fragment of any of claims 1-3, **characterized in** comprising
- the heavy chain variable domains
- CDR3 region having an amino acid sequence being at least 90% identical to said in SEQ ID NO: 35 and/or
- CDR2 region having an amino acid sequence being at least 90% identical to said in SEQ ID NO: 34 and/or
- CDR1 region having an amino acid sequence being at least 90% identical to said in SEQ ID NO: 33 and/or
- the light chain variable domains
- CDR3 region having an amino acid sequence being at least 90% identical to said in SEQ ID NO: 38 and/or
- CDR2 region having an amino acid sequence being at least 90% identical to said in SEQ ID NO: 37 and/or
- CDR1 region having an amino acid sequence being at least 90% identical to said in SEQ ID NO: 36.

7. The antibody of fragment thereof or any of the preceding claims comprising
- a heavy chain having an amino acid sequence being at least 90% identical to said in SEQ ID NO: 11, and/or
- a light chain having an amino acid sequence being at least 90% identical to said in SEQ ID NO: 16.

8. The antibody of fragment thereof or any of the preceding claims comprising
- a heavy chain having an amino acid sequence being at least 90% identical to said in SEQ ID NO: 27, and/or
- a light chain having an amino acid sequence being at least 90% identical to said in SEQ ID NO: 32.

9. The antibody of fragment thereof or any of the preceding claims comprising
- a heavy chain having an amino acid sequence being at least 90% identical to said in SEQ ID NO: 43, and/or
- a light chain having an amino acid sequence being at least 90% identical to said in SEQ ID NO: 48.

10. An antibody or fragment thereof configured for a specific binding to glycoprotein 100 (gp100) for use in the prophylaxis and/or treatment of cancer, preferably gp100-expressing tumors, highly preferably melanoma.

11. The antibody or fragment thereof of claim 10, which is the antibody or fragment thereof of any of claims 1-9.

12. A pharmaceutical composition **characterized in** comprising an antibody or fragment thereof according to any of claims 1-11.

13. A nucleic acid encoding an antibody or fragment thereof according to any of claims 1-12.

14. A vector comprising the nucleic acid of claim 13 and, optionally, regulatory elements necessary for an expression in a prokaryotic and/or eukaryotic cell.

15. A prokaryotic or eukaryotic host cell comprising the vector of claim 14.
